# EUROPEAN PATENT SPECIFICATION

(11) **EP 4 193 343 B1**
(45) Date of publication and mention of the grant of the patent: **12.03.2025**
(21) Application number: 21765709.7
(22) Date of filing: 06.08.2021
(51) Int. Cl.: G07F 19/00, A61L 2/10

(54) **AUTOMATIC OBJECT/ DATA DISPENSING/ INSERTION SYSTEM**
AUTOMATISCHES SYSTEM ZUM AUSGEBEN/EINBRINGEN VON GEGENSTÄNDEN/DATEN
SYSTÈME AUTOMATIQUE DE DISTRIBUTION/INSERTION D'OBJETS/DONNÉES

(30) Priority: 10.08.2020 IT 202000019861
(43) Date of publication of application: 14.06.2023
(73) Proprietor: ATM Lab S.r.l., 27026 Garlasco (Pavia) (IT); Cristofaro, Domenico, 89017 San Giorgio Morgeto (Reggio Calabria) (IT)
(72) Inventor: MORANO, Domenico, 27026 Garlasco (Pavia) (IT); CRISTOFARO, Domenico, 89017 San Giorgio Morgeto (Reggio Calabria) (IT)
(74) Representative: Praxi Intellectual Property Milano
(86) International application number: PCT/IB2021/057254
(87) International publication number: WO 2022/034454

(56) References cited:
- WO-A1-2015/051024
- CN-A- 109 961 557
- KR-B1- 101 833 319
- US-A1- 2011 256 019
- US-A1- 2018 339 075

## Description

### FIELD OF THE TECHNIQUE

The present invention relates to automatic object/data dispensing/insertion systems such as, but not limited to, ATMs.

### STATE OF THE ART

The ATM (Automatic Teller Machine) is, as is well known, an automatic machine that allows for operations such as withdrawing cash, requesting account information and, if enabled, depositing cash or cheques and making transfers.

Other known dispensers include, for example, beverage or packaged food dispensers, cigarette dispensers, and ticket dispensers.

The situation related to the pandemic of Covid-19 (also called SARS-CoV -2) or similar has highlighted how this type of device can be a dangerous vehicle for the spread of bacteria or viruses. In fact, their use involves the manual contact of the user with the device itself (think of a keyboard or a touch screen, but not only), in effect the proximity of the user can lead to the dispersion on parts of the device of viruses/bacteria present on the hands or in respiratory droplets that can escape from the mouth and nose of the user.

The above-mentioned risks do not concern only the users, but also the operators who work on such apparatuses to carry out maintenance, withdrawal of money and/or refill of the item to be dispensed.

The mask and the gloves worn (not always) by the user and also the disinfection of the hands do not constitute real solutions to the above-mentioned problem since there is no certainty on the actual behavior of the users, also considering the fact that these precautions certainly involve a nuisance.

US document 2011/0256019 describes a device for sterilizing a touch-type display that includes a source of ultraviolet light, posterior to the display. A screen is also provided to protect users from the ultraviolet light.

### SUMMARY OF THE INVENTION

It is the purpose of the present invention to propose a system comprising a plurality of automatic object/data dispensing/insertion devices that can be used safely for users or operators in relation to the spread of contagious diseases.

Applicants have perceived that a solution to the above problem may lie in the use of an electromagnetic sanitizing radiation that entirely invades the area from which said automatic devices are accessible, coupled with a movable screen that inhibits access to said area, and thus the use of the dispensing/insertion system during the sanitizing action performed by the electromagnetic radiation.

It is an object of the present invention to provide a dispensing/insertion system as defined by claim 1 and particular embodiments thereof described by dependent claims 2-13.

### BRIEF DESCRIPTION OF THE DRAWINGS

The constructional and functional features of the invention may be better understood from the following detailed description, in which reference is made to the accompanying drawing plates representing some preferred and non-limiting forms of embodiment thereof, wherein:
- figure 1 shows a perspective view of a system for the dispensing/insertion of objects/data that is realized, according to an example, in an ATM system, represented in an access configuration, i.e. with a raised mobile screen up;
- figure 2 shows said ATM system, in a front view, in the access configuration, i.e. with the movable screen up;
- figure 3 shows said ATM system in a anterior front view, represented in a protection configuration, that is with the movable screen down;
- Figure 4 shows said ATM system in a perspective view, in the protection configuration, i.e. with the movable screen lowered.

### DETAILED DESCRIPTION

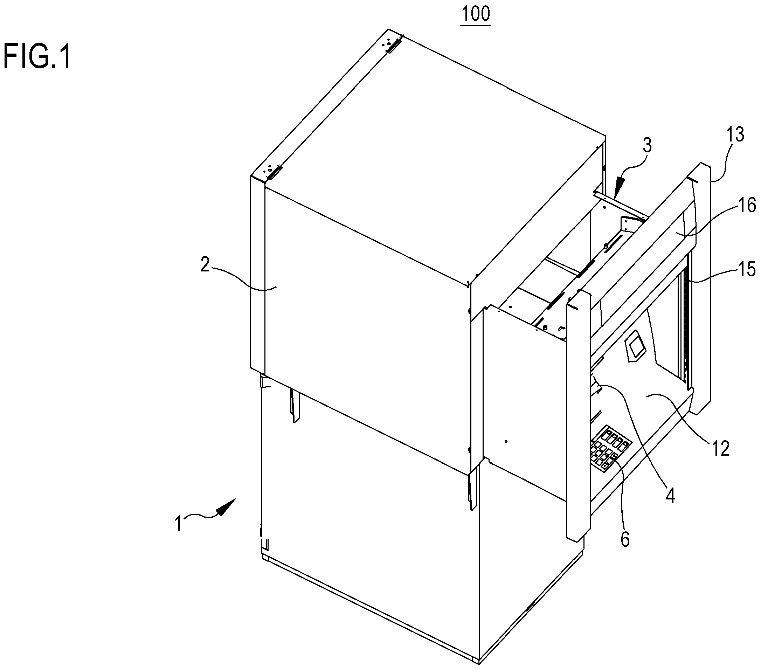

Figures 1-4 illustrate, by way of example, an Automatic Teller Machine (ATM) system 100 implemented in accordance with one embodiment of the present invention.

As is well known, an ATM (also known by the term "ATM"), is an automated device configured to perform operations such as: withdrawing cash, requesting account information, depositing cash or checks, making wire transfers, making payments (e.g., fines, utilities, phone refills, etc.).

Before continuing with the description of ATM 100, it should be noted that the teachings of this description are not only applicable to ATMs but are also applicable to other types of automatic systems for dispensing/inserting objects/data such as, for example: ticket vending machines, reservation/shift number dispensers in public or private offices, vending machines for articles (e.g.: cigarettes, drugs, medical devices, packaged foods or beverages, etc.).

In particular, for the purposes of the present invention, automatic dispensing/insertion object/data systems are also understood to include enabling systems that (e.g., after data, money, credit cards or tickets are entered) activate a given operation. For example, devices for enabling/activating self-service fuel withdrawal can be considered particular automatic dispensing/inserting objects/data systems.

In addition, the technical teachings described herein are also applicable to automated teller machines such as highway toll booths or other automated apparatus where physical contact occurs between a user and some of the electronic (e.g., a keypad) or electromechanical devices (e.g., a dispensing or object insertion port).

With reference to the ATM 100 shown in Figures 1-4, it comprises a support structure 1 including, for example: a container 2 and a user interaction structure 3.

The container 2 houses, among other things, dispensing apparatuses that preside over the withdrawal and delivery of money (or the distribution/receipt of documents). Container 2, which may possibly be housed in an internal compartment (with controlled access) of a building or a booth, also has the important function of protection against burglary or theft attempts, especially when the ATM faces directly outside, without other protections /obstructions.

A device for controlling the operation of the ATM 100 may be housed in the enclosure 2.

The user interaction structure 3 includes at least one electronic/mechanical object/data dispensing/insertion device.

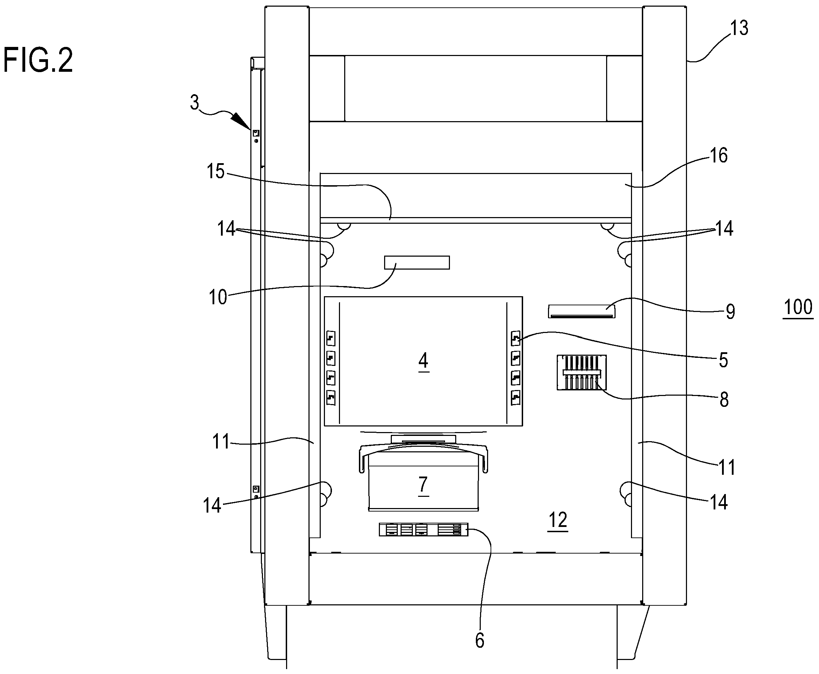

In particular, with respect to data entry the user interaction structure 3 may be provided (Figure 2) with a touch screen 4 optionally provided with selection buttons 5. For example, the user interaction structure 3 may further comprise an alphanumeric keyboard 6.

In addition, there is provided a banknote dispensing mouth 7 (so-called, "shutter") equipped, for example, with a movable door and from which the user can manually take the banknotes that are proposed to be dispensed. According to some embodiments, the same shutter 7 may be used to insert banknotes to be paid into an account.

For example, the user interaction structure 3 is also provided with a slot 8 for inserting credit or ATM cards (associated with a suitable reader, not shown) and, for example, a receipt dispenser 9 (associated with a related printer, not shown). A check insertion slot 10 for depositing checks may also be provided, subject to scanning.

For dispensing/insertion systems other than the ATM, other examples of electronic/mechanical dispensing/insertion devices for objects/data are: a ticket insertion mouth, a bill insertion/return mouth, a coin insertion/return mouth, a product dispensing mouth (e.g., tickets, food, beverages, cigarettes, drugs, etc.).

Returning to the ATM 100, the electronic/mechanical dispensing/insertion object/data devices (4-10) described above are arranged to be accessible on an interaction panel 12 that is bounded by a frame or frame structure 13. For example, but not limited to, the frame structure 13 (e.g., metal) is embedded in an opening in a masonry wall.

It should be noted that the ATM 100 may also have a different structure than depicted in the figures. For example, the ATM 100 may be a Through-The-Wall (TTW) type as depicted in the figures or a Lobby type.

The ATM 100 is further provided with at least one sanitizing electromagnetic radiation source such as, preferably, one or more germicidal ultraviolet radiation sources advantageously configured to generate ultraviolet light with wavelengths in the UV-C band (100-280 nm).

In an embodiment not covered by the claims, such a radiation source is mounted on the support structure 1 and is arranged to invest those electronic/mechanical devices (devices 4-10 in the figures) with at least part of the support structure 1 (e.g., the interaction panel 12).

In particular, these sources take the form of one or more UV-C lamps 14 (which may be of commercially available types) arranged to invest the interaction panel 12 and thereby all electronic/mechanical devices 4-10. Such UV-C lamps 14 are only schematically depicted as light points in the accompanying figures. For example, the UV-C lamps 14 may be of the LED type.

In particular, one or more UV-C lamps 14 (e.g., at least six lamps) are placed on the frame structure 13 and oriented to invest at least the interaction panel 12. The UV-C lamps 14 are mounted, for example, on jambs 11 and/or on a lintel 16 of the frame structure 13. Within the frame structure 13, according to the example, power circuitry for the UV-C sources 14 may be suitably housed.

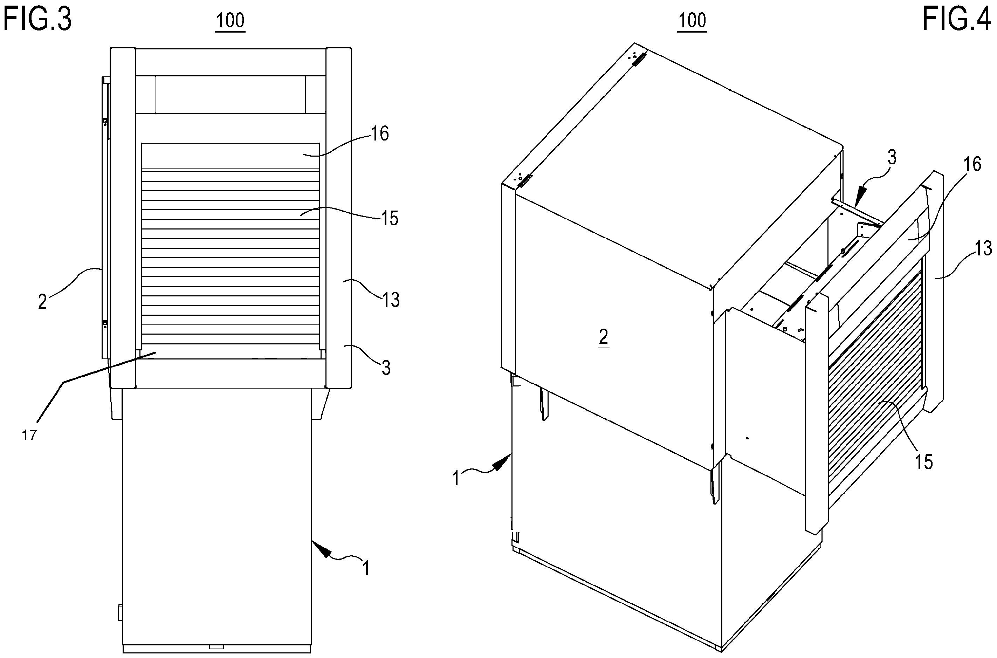

The ATM 100 is further provided with a movable screen 15 associated with the support structure 1 and configured to selectively assume an access position (Figures 1 and 2) and a protection position (Figures 3 and 4) in which, respectively, it enables and disables user access to the electronic/mechanical devices 4-10 and the interaction panel 12.

Advantageously, the mobile screen 15 advantageously operates as both a physical barrier to user access to the interaction panel 12 and as a radiation shield as it is opaque to UV-C radiation. Advantageously, the mobile screen 15 is also watertight. In particular, the movable screen 15 is a shutter (e.g., rolling shutter), as depicted in the figures, but may also be of another type such as, for example, a curtain or a movable door.

The rolling shutter 15 is formed of a plurality of laths bonded together (made of metal or plastic, or other suitable material) and in the access configuration (open) is rolled up and collected, for example, in an area inside the lintel 16 of the frame structure 13. For example, the laths of the rolling shutter 15 may be made of insulated aluminum.

Further, according to the claimed embodiment, at least one UV-C lamp 14 (in
particular, in the form of LEDs), or all of those employed, are mounted on an inner face of the rolling shutter 15. For example, the UV-C LEDs 14 are mounted on an terminal member 17 of the rolling shutter 15 (also referred to herein as an end piece), such as an terminal lath of the free portion. This terminal lath 17 has a greater weight than the other laths since it must facilitate the lowering of the rolling shutter during closing.

The movement of the rolling shutter 15 between the access position (raised, i.e. open) and the protection position (lowered, i.e. closed) takes place by means of a suitable electric motor (not shown) housed, for example, in the frame structure 13. According to the example, the rolling shutter 15 moves between the two positions by sliding within side guides provided in the frame structure 13.

In the protection position (and thus sanitization in progress), the rolling shutter15 is fully lowered so as to close and prevent access for the user to the area of the interaction panel 12 and thus to the various devices/mechanics 4-10. In the protective position, the UV-C lamps 14 are arranged in an inner zone between the rolling shutter 15 and the interaction panel 12.

The rolling shutter 15 also has the advantage that, in the protective position, it performs an anti-effraction function of the ATM 100.

The electric motor and UV-C lamps 14 are controlled by a control module (not shown) in communication with, or coincident with, the control device of the ATM 100. The control module (a computer, such as a microprocessor, housed, for example, in the container 2) operates according to a predetermined management procedure, which takes into account both the need for sanitization of the interaction panel 12 and the need to prevent the user from being exposed to radiation emitted by the UV-C lamps 14.

Note that the movable screen 15 and/or the electric motor and/or the UV-C lamps 14 may be mounted on a structure separate from the interaction panel 12 such as, for example, a masonry structure surrounding the interaction panel 12. With reference to an example of operation, consider a user approaching the ATM 100 to perform an operation. According to this example, the shutter 15 is wrapped within the lintel 16 and there is full access to the interaction panel 12.

The user performs the necessary operations by touching with his or her hands at least some of the electronic/mechanical devices 4-10 and probably the interaction panel 12. There is, of course, the further possibility that the user himself or herself disperses pathogens (such as bacteria or viruses) on the panel 12 that are present in respiratory droplets and aerosols that may leak from the mouth and nose.

When the user has completed the operations this is detected by the control module of the ATM 100 and then by the sanitization control device which can activate a sanitization cycle or procedure.

Preferably, the ATM 100 may be equipped with sensors (for example, photocells), in communication with the control device, which detect the presence of the user at the ATM 100: if the user fails to leave, the control device interrupts or delays the sanitization cycle.

In the absence of obstacles, the sanitizing cycle is enabled. This cycle comprises the activation of the electric motor that causes the lowering of the roller shutter 15 until it assumes the protection position. Prior to and/or during such movement of the roller shutter 15, audible alerts (e.g., a recorded voice message) or forms of visual alerts informing that lowering of the roller shutter 15 is occurring may advantageously be activated.

The lowering of the rolling shutter 15 can be interrupted if special sensors (for example, the aforementioned photocells or electromechanical sensors connected to the shutter 15), in communication with the control device, detect the presence of obstacles (for example, the hand of a user or forgotten objects) that prevent the shutter 15 from closing properly. In an embodiment not covered by the claims, when the shutter 15 is fully lowered, the control device activates the UV-C lamps 14 which generate the radiation that invests the interaction panel 12 and the electronic/mechanical devices 4-10 carrying out the sanitization.

According to the claimed embodiment,
the sanitization by the UV-C lamps 14 takes place during the movement of the shutter 15 from the access configuration to the protection configuration and/or vice versa. This may preferably occur if one or more UV_C LEDs 14 are mounted on the terminal lath 17. In this case, during the movement of the rolling shutter 15, the UV-C LED 14 is activated so as to carry out a sanitization of each zone of the panel 12 that it faces (i.e., opposes) during the movement.

In particular, the keyboard 6 and the touch screen 4 with which there is manual contact are sanitized. To make this sanitization treatment more complete, the controlled opening of the mobile door associated with the banknote dispensing mouth 7 can also be envisaged in order to carry out sanitization on the inside.

Sanitization by means of UV-C radiation is carried out for an established time interval, compatible with the current average time of use of the ATMs by each individual user. The movement time of the roller shutter 15 can be reduced to a few seconds: for example, between the protection position and the access position, and vice versa, a time of less than 5.00 s or preferably, less than 3.00 s elapses (for example, equal to about 2.00 s).

During sanitization, the rolling shutter 15 prevents access to the interaction panel 12 and also does not allow UV-C radiation (direct or reflected) to run over users or passersby who may be in the vicinity of the ATM 100.

At the end of this time interval, the control device interrupts the generation of UV-C radiation (i.e., turns off the lamps 14) and then activates the electric motor that brings, by winding, the shutter 15 to the access position, making the interaction panel 12 available again for use by a subsequent user or operator (e.g., a maintenance person).

The sanitization cycle described above is repeated, preferably, at each change of user.

The solution described is very advantageous in that it offers the possibility of full use of automatic systems for dispensing/inserting objects/data, avoiding or considerably reducing the risk of contagion from easily transmitted infectious diseases, without requiring human intervention for sanitization.

Moreover, the presence of an opaque mobile screen eliminates any risk for the user of being hit by germicidal radiation.

The solution described is advantageous not only for the users of the dispensing/insertion systems to which it is applicable, but also for the operators who work on such systems to perform, for example, maintenance, cleaning, withdrawal of money and/or refilling of the item to be dispensed.

It should also be noted that the solution described can be implemented both on automated systems specifically designed to provide for it, and on machines already in use, since the addition of the components necessary to implement it does not appear particularly complex.

### Legend of drawing number references

- ATM system 100
- support structure 1
- container 2
- user interaction structure 3
- touchscreen 4
- selection buttons 5
- keyboard 6
- banknote dispensing/insertion slot 7 ("shutter")
- slot for credit or debit card insertion 8
- receipt dispenser 9
- cheque insertion slot 10
- jambs 11
- interaction panel 12
- frame structure 13
- UV-C lamps 14
- 15 movable screen (rolling shutter)
- lintel 16
- terminal lath 17

## Claims

1. An automated object/data dispensing/insertion system (100) comprising:
- a support structure (1) comprising a frame structure (13);
- an interaction panel (12) delimited by said frame structure (13);
- a plurality of electronic/mechanical object/data dispensing/insertion devices (4-10) mounted on the support structure (1), arranged to be accessible from the interaction panel (12) and configured to operate by manual contact of a user of the system;
- at least one sanitizing electromagnetic radiation source (14) arranged to irradiate the
interaction panel (12) and at least some of the electronic/mechanical devices (4-10);
- a movable screen (15) associated with the frame structure (13) of the support
structure (1) and configured to selectively assume an access position and a protection position in which, respectively, it enables and disables user access to the interaction panel (12) and thus to the electronic/mechanical devices (4-10); said at least one source being such that it is disposed, in the protection position, in an internal zone between the movable screen (15) and the interaction panel (12);
- a) a control device of the radiation source (14) configured to: activate the radiation source (14)
subsequent to a use of the system by a user and for a sanitization time interval; deactivate the radiation source (14) at the end of that time interval;
- b) an electric motor controlled by the control device, mechanically coupled to the movable screen (15) and configured to move the movable screen between the protective position and the access position;
**characterized in that:**
c) the at least one sanitizing electromagnetic radiation source (14) is mounted on an inner face of said movable screen (15), and
d) said control device is configured to activate the radiation source (14) when the electric motor moves the movable screen between the protective position and the access position and/or vice versa.

2. System (100) according to claim 1, wherein said at least one sanitizing radiation source (14) is a germicidal ultraviolet radiation source configured to generate ultraviolet light having wavelengths within the UV-C band.

3. System (100) according to claim 1, wherein said at least one sanitizing radiation source (14) comprises a plurality of UV-C lamps.

4. System (100) according to claim 1, wherein said movable screen (15) is opaque to said sanitization radiation.

5. System (100) according to claim 1, wherein said movable screen (15) is one of the following devices: a rolling shutter, a curtain, a movable door.

6. System (100) according to claim 5, wherein said movable screen (15) is a rolling shutter comprising a plurality of bonded laths and said at least one sanitizing electromagnetic radiation source (14) is mounted on a terminal portion of the rolling shutter.

7. System (100) according to claim 1, wherein said electronic/mechanical object/data dispensing/insertion devices include at least one of the following: Manual data entry keyboard (6), touch screen (4) for data entry, object dispensing mouth (9), sales receipt and/or receipt dispensing mouth (9), bill dispensing mouth (7), electronic card reader (8), check deposit insertion slot (10), ticket insertion mouth, bill insertion/return mouth, coin insertion/return mouth.

8. System (100) according to claim 1, further comprising at least one sensor device in communication with the control device and configured to detect the presence of obstacles to the movement of the movable screen (5) between the access position and the protection position; the control device is further configured to block the movement of the movable screen (5) when the presence of the obstacles to the movement of the movable screen (5) is detected.

9. System (100) according to claim 1, wherein said system is one of the following apparatus: an ATM apparatus, a ticket vending machine, a device for enabling/activating self-service fuel; a reservation/shift number dispenser in public or private offices, a vending machine for articles, a beverage dispenser, a medication dispenser, a packaged food dispenser.

10. System according to claim 1, wherein said movable screen (15) is configured with burglary-resistant features.

11. System according to claim 1, wherein said control device and said electric motor are configured to move said movable screen (15) between the protection position and the access position and vice versa in a time of less than 5.00 s, preferably, less than 3.00 s.

12. System according to claim 1, wherein said electric motor is a 24V motor.

13. System according to claim 1, wherein:
- said radiation source (14) comprises at least one LED and is mounted on a portion of said movable screen (15) facing the panel (12).

## Patentansprüche

1. Automatisiertes Objekt-/Datenausgabe-/Eingabesystem (100), umfassend:
- eine Trägerstruktur (1), umfassend eine Rahmenstruktur (13);
- ein Interaktionsfeld (12), das durch die Rahmenstruktur (13) begrenzt ist;
- eine Vielzahl von elektronischen/mechanischen Objekt-/Datenausgabe- /Eingabevorrichtungen (4-10), die auf der Trägerstruktur (1) montiert sind, angeordnet, um vom Interaktionspanel (12) aus zugänglich zu sein, und konfiguriert sind, um durch manuellen Kontakt eines Benutzers des Systems bedient werden zu können;
- mindestens eine desinfizierende elektromagnetische Strahlungsquelle (14), angeordnet, um das Interaktionspanel (12) und mindestens einige der elektronischen/mechanischen Vorrichtungen (4-10) zu bestrahlen;
- einen beweglichen Bildschirm (15), der mit der Rahmenstruktur (13) der Trägerstruktur (1) verbunden ist und konfiguriert ist, um selektiv eine Zugriffsposition und eine Schutzposition einzunehmen, in denen er dem Benutzer den Zugriff auf das Interaktionspanel (12) und somit auf die elektronischen/mechanischen Vorrichtungen (4-10) ermöglicht bzw. sperrt; wobei die mindestens eine Quelle so beschaffen ist, dass sie in der Schutzposition in einer Innenzone zwischen dem beweglichen Bildschirm (15) und dem Interaktionspanel (12) angeordnet ist;
- a) eine Steuervorrichtung der Strahlungsquelle (14), die konfiguriert ist zum:
Aktivieren der Strahlungsquelle (14) nach einer Verwendung des Systems durch einen Benutzer und für ein Desinfektionszeitintervall;
Deaktivieren der Strahlungsquelle (14) am Ende dieses Zeitintervalls;
- b) einen von der Steuervorrichtung gesteuerten Elektromotor, der mechanisch mit dem beweglichen Schirm (15) gekoppelt und konfiguriert ist, um den beweglichen Schirm zwischen der Schutzposition und der Zugangsposition zu bewegen;
**dadurch gekennzeichnet, dass:**
c) die mindestens eine desinfizierende elektromagnetische Strahlungsquelle (14) an einer Innenfläche des beweglichen Schirms (15) angebracht ist, und
d) die Steuervorrichtung konfiguriert ist, um die Strahlungsquelle (14) zu aktivieren, wenn der Elektromotor den beweglichen Schirm zwischen der Schutzposition und der Zugangsposition und/oder umgekehrt bewegt.

2. System (100) nach Anspruch 1, wobei die mindestens eine desinfizierende Strahlungsquelle (14) eine keimtötende Ultraviolettstrahlungsquelle ist, die konfiguriert ist, um Ultraviolettlicht mit Wellenlängen innerhalb des UV-C-Bands zu erzeugen.

3. System (100) nach Anspruch 1, wobei die mindestens eine desinfizierende Strahlungsquelle (14) eine Vielzahl von UV-C-Lampen umfasst.

4. System (100) nach Anspruch 1, wobei der bewegliche Schirm (15) für die Desinfektionsstrahlung undurchlässig ist.

5. System (100) nach Anspruch 1, wobei der bewegliche Schirm (15) eine der folgenden Vorrichtungen ist: ein Rollladen, ein Vorhang, eine bewegliche Tür.

6. System (100) nach Anspruch 5, wobei der bewegliche Schirm (15) ein Rollladen ist, der eine Vielzahl verbundener Latten umfasst, und wobei die mindestens eine desinfizierende elektromagnetische Strahlungsquelle (14) an einem Endabschnitt des Rollladens angebracht ist.

7. System (100) nach Anspruch 1, wobei die elektronischen/mechanischen Vorrichtungen zum Ausgeben/Eingeben von Objekten/Daten mindestens eines der folgenden umfassen: Tastatur (6) für die manuelle Dateneingabe, Berührungsbildschirm (4) zur Dateneingabe, Objektausgabeöffnung (9), Ausgabeöffnung für Verkaufsbelege und/oder Quittungen (9), Geldscheinausgabeöffnung (7), elektronisches Kartenlesegerät (8), Einwurfschlitz für Scheckeinzahlungen (10), Fahrkarteneinstecköffnung, Geldscheineinwurf-/- rückgabeöffnung, Münzeinwurf-/-rückgabeöffnung.

8. System (100) nach Anspruch 1, ferner umfassend mindestens eine Sensorvorrichtung, die mit der Steuervorrichtung in Verbindung steht und konfiguriert ist, um das Vorhandensein von Hindernissen für die Bewegung des beweglichen Bildschirms (5) zwischen der Zugangsposition und der Schutzposition zu erkennen; die Steuervorrichtung ist ferner konfiguriert, um die Bewegung des beweglichen Bildschirms (5) zu blockieren, wenn das Vorhandensein der Hindernisse für die Bewegung des beweglichen Bildschirms (5) erkannt wird.

9. System (100) nach Anspruch 1, wobei das System eines der folgenden Vorrichtungen ist: ein Geldautomat, ein Fahrkartenautomat, ein Gerät zum Freigeben/Aktivieren von Selbstbedienungstankstellen; ein Reservierungs- /Wartenummernautomat in öffentlichen oder privaten Büros, ein Verkaufsautomat für Artikel, ein Getränkeautomat, ein Medikamentenautomat, ein Automat für abgepackte Nahrungsmittel.

10. System nach Anspruch 1, wobei der bewegliche Bildschirm (15) mit einbruchhemmenden Merkmalen ausgestattet ist.

11. System nach Anspruch 1, wobei die Steuervorrichtung und der Elektromotor konfiguriert sind, um den beweglichen Schirm (15) in einer Zeit von weniger als 5,00 s, vorzugsweise weniger als 3,00 s, zwischen der Schutzposition und der Zugangsposition und umgekehrt zu bewegen.

12. System nach Anspruch 1, wobei der Elektromotor ein 24-V-Motor ist.

13. System nach Anspruch 1, wobei:
- die Strahlungsquelle (14) mindestens eine LED umfasst und auf einem dem Panel (12) zugewandten Teil des beweglichen Bildschirms (15) montiert ist.

## Revendications

1. Système automatisé de distribution/insertion d'objets/de données (100) comprenant :
- une structure de support (1) comprenant une structure de cadre (13) ;
- un panneau d'interaction (12) délimité par ladite structure de cadre (13) ;
- une pluralité de dispositifs électroniques/mécaniques de distribution/insertion d'objets/de données (4-10) montés sur la structure de support (1), agencés de manière à être accessibles depuis le panneau d'interaction (12) et configurés pour fonctionner par contact manuel d'un utilisateur du système ;
- au moins une source de rayonnement électromagnétique d'assainissement (14) agencée pour irradier le panneau d'interaction (12) et au moins certains des dispositifs électroniques/mécaniques (4-10) ;
- un écran mobile (15) associé à la structure de cadre (13) de la structure de support (1) et conçu pour prendre sélectivement une position d'accès et une position de protection dans lesquelles, respectivement, il active et désactive l'accès de l'utilisateur au panneau d'interaction (12) et donc aux dispositifs électroniques/mécaniques (4-10) ; ladite au moins une source étant telle qu'elle est disposée, en position de protection, dans une zone interne entre l'écran mobile (15) et le panneau d'interaction (12) ;
- a) un dispositif de commande de la source de rayonnement (14) configuré pour :
activer la source de rayonnement (14) après une utilisation du système par un utilisateur et pendant un intervalle de temps d'assainissement ;
désactiver la source de rayonnement (14) à la fin de cet intervalle de temps ;
- b) un moteur électrique commandé par le dispositif de commande, couplé mécaniquement à l'écran mobile (15) et configuré pour déplacer l'écran mobile entre la position de protection et la position d'accès ;
**caractérisé en ce que** :
c) l'au moins une source de rayonnement électromagnétique d'assainissement (14) est montée sur une face interne dudit écran mobile (15), et
d) ledit dispositif de commande est configuré pour activer la source de rayonnement (14) lorsque le moteur électrique déplace l'écran mobile entre la position de protection et la position d'accès et/ou vice versa.

2. Système (100) selon la revendication 1, dans lequel ladite au moins une source de rayonnement d'assainissement (14) est une source de rayonnement ultraviolet germicide configurée pour générer une lumière ultraviolette dont les longueurs d'onde se situent dans la bande UV-C.

3. Système (100) selon la revendication 1, dans lequel ladite au moins une source de rayonnement d'assainissement (14) comprend une pluralité de lampes UV-C.

4. Système (100) selon la revendication 1, dans lequel ledit écran mobile (15) est opaque audit rayonnement d'assainissement.

5. Système (100) selon la revendication 1, dans lequel ledit écran mobile (15) est l'un des dispositifs suivants : un volet roulant, un rideau, une porte mobile.

6. Système (100) selon la revendication 5, dans lequel ledit écran mobile (15) est un volet roulant comprenant une pluralité de lattes liées et ladite au moins une source de rayonnement électromagnétique d'assainissement (14) est montée sur une partie d'extrémité du volet roulant.

7. Système (100) selon la revendication 1, dans lequel lesdits dispositifs électroniques/mécaniques de distribution/insertion d'objet/de données comportent au moins l'un de ce qui suit : un clavier de saisie de données manuelle (6), un écran tactile (4) pour la saisie des données, une ouverture de distribution d'objets (9), une ouverture de distribution de tickets de caisse et/ou de reçus (9), une ouverture de distribution de billets (7), un lecteur de cartes électroniques (8), une fente d'insertion de dépôt de chèques (10), une ouverture d'insertion de tickets, une ouverture d'insertion/retour de billets, une ouverture d'insertion/de retour de pièces de monnaies.

8. Système (100) selon la revendication 1, comprenant en outre au moins un dispositif de détection en communication avec le dispositif de commande et configuré pour détecter la présence d'obstacles au mouvement de l'écran mobile (5) entre la position d'accès et la position de protection ; le dispositif de commande est en outre configuré pour bloquer le mouvement de l'écran mobile (5) lorsque la présence des obstacles au mouvement de l'écran mobile (5) est détectée.

9. Système (100) selon la revendication 1, dans lequel ledit système est l'un des appareils suivants : un appareil de guichet automatique bancaire, une machine distributrice de tickets, un dispositif permettant d'activer le carburant en libre-service ; un distributeur de réservation/numéro d'équipe dans des bureaux publics ou privés, une machine distributrice d'articles, un distributeur de boissons, un distributeur de médicaments, un distributeur d'aliments emballés.

10. Système selon la revendication 1, dans lequel ledit écran mobile (15) est configuré avec des caractéristiques anti-effraction.

11. Système selon la revendication 1, dans lequel ledit dispositif de commande et ledit moteur électrique sont configurés pour déplacer ledit écran mobile (15) entre la position de protection et la position d'accès et vice versa en un temps inférieur à 5,00 s, de préférence inférieur à 3,00 s.

12. Système selon la revendication 1, dans lequel ledit moteur électrique est un moteur 24 V.

13. Système selon la revendication 1, dans lequel :
- ladite source de rayonnement (14) comprend au moins une DEL et est montée sur une partie dudit écran mobile (15) orientée vers le panneau (12).
